(19) **Europäisches Patentamt**
**European Patent Office**
**Office européen des brevets**

(11) **EP 2 388 723 A1**

(12) **EUROPEAN PATENT APPLICATION**

(43) Date of publication:
**23.11.2011 Bulletin 2011/47**

(51) Int Cl.:
**G06F 19/00** $^{(2011.01)}$

(21) Application number: **10163132.3**

(22) Date of filing: **18.05.2010**

(84) Designated Contracting States:
**AL AT BE BG CH CY CZ DE DK EE ES FI FR GB GR HR HU IE IS IT LI LT LU LV MC MK MT NL NO PL PT RO SE SI SK SM TR**
Designated Extension States:
**BA ME RS**

(71) Applicant: **University College Cork-National University of Ireland, Cork**
**Cork (IE)**

(72) Inventors:
- **DunnGalvin, Audrey**
  **Cork (IE)**
- **Hourihane, Johnathan O'B.**
  **Cork (IE)**

(74) Representative: **Purdy, Hugh Barry**
**PurdyLucy**
**Intellectual Property**
**Suite 138/139**
**The Capel Building**
**Mary's Abbey**
**Dublin 7 (IE)**

(54) **Method of assessing allergic status in a subject**

(57)    A method of assessing the allergic status of a subject to a specific allergen, comprises the steps of determining a subject-specific numeric value for a plurality of variables comprising age, sex, SPT, IgE, (sIgE or tIgE minus sIgE) and allergic symptoms, applying an allergen-specific weighting to the numeric value for each variable, and correlating the weighted numeric values with allergic status for the subject. The weighted numeric values may be correlated with allergenicity status using a linear regression model $P = ez/(1= ez)$ in which: P = predicted allergenicity status for the subject; e = mathematical constant (2.71828....); and z = linear combination of the weighted numeric values for the variables.

EP 2 388 723 A1

**Description**

The invention relates to a method of assessing allergic status in a subject. In particular, the invention provides a method of predicting the outcome of oral food challenge in a subject.

Food allergy is an immune system reaction that occurs soon after eating a certain food. Even a tiny amount of the allergy-causing food can trigger signs and symptoms such as digestive problems, hives or swollen airways. In some people, a food allergy can cause severe symptoms or even a life-threatening reaction known as anaphylaxis. Tree nuts and peanuts are the leading causes of deadly allergic reactions called anaphylaxis.

Food allergy affects an estimated 6 to 8 percent of children under age 3, and about 4 percent of adults. While there's no cure, some children outgrow their food allergy as they get older. In adults, the foods that most often trigger allergic reactions include fish and shellfish, such as shrimp, lobster and crab, peanuts, tree nuts, such as walnuts, and eggs. Problem foods for children include eggs, milk (especially in infants and young children), and peanuts.

The last decade has shown both an increasing prevalence of food allergy and increasing numbers of patients and parents seeking diagnosis. While double-blind, placebo-controlled food challenges (DBPCFC) still represent the gold standard for the diagnosis of food allergy, they are time-consuming and expensive and they create some concerns for patients and parents, for example the fear and risk of severe systemic reactions. Many paediatric units routinely use open food challenges (OFC) outside research protocols. In addition, not all clinical locations have the facilities to carry out high quality food challenges. Given these factors, efforts have been made to find diagnostic tests to predict the outcome of oral food challenges. These prognostic models have focused on decision points for sIgE or SPT, either independently or in combination resulting in predictive values of 61% - 81%, which although useful, are not high enough for confident predictions of oral food challenges.

Furthermore, a history of anaphylaxis is often used to decide not to offer a food challenge although resolution of food allergy is still possible with such a history. So it appears other clinical factors are inconsistently considered and their impact on challenge outcome has previously never been evaluated systematically, although they are likely to be influential. In addition, when a patient falls within an immunological grey area (i.e. below the 95% cut off point for 'certain reactivity' but above a level that would predict a negative outcome, usually a completely negative SPT or sIgE), there has not as yet been any method developed to confidently choose which of the two potential outcomes is most likely.

It is an object of the invention to overcome at least one of the above-referenced problems.

Statements of Invention

The invention broadly relates to a method of assessing the allergic status of a subject, typically to a specific allergan, for example (pea)nut, milk or egg. The term "allergic status" should be understood to mean assessing or predicting whether the subject is allergic to the specific allergen, whether the subject is at risk of developing an allergy to a specific allergan, whether the subject is responding to a therapy aimed at alleviating or curing a food-related allergic reaction, determining a stop/continue point during a maintenance phase in oral immunotherapy, or determining whether an allergic response to a specific allergan has recurred in a subject. In a particular embodiment, the term should be understood to mean predicting the outcome of oral food challenge in a subject.

The method employs a number of subject-specific variables which are easily obtained from the subject, wherein the combination of variables may be correlated with risk of an allergic reaction to the specific allergen. The method of the invention provides higher sensitivity and specificity than existing methods of predicting risk of allergic reaction such as specific sIgE, SPT, or sIgE in combination with SPT. The method involves determining a value for the age, sex, STP, IgE (for example specific IgE (sIgE) or total minus specific IgE (tIgE-sIgE)), and symptoms, for the subject, applying an allergan-specific weighting to the values, and correlating the weighted values with risk of the subject being allergic to the specific allergen, typically by means of a linear regression model.

Accordingly, the invention relates to a method of assessing the allergic status of a subject to a specific allergen, the method comprising the steps of determining a subject-specific numeric value for a plurality of variables comprising age, sex, SPT, IgE, and allergic symptoms, applying an allergen-specific weighting to the numeric value for each variable, and correlating the weighted numeric values with allergic status for the subject.

In one embodiment, the IgE is specific IgE (sIgE). In a preferred embodiment, two IgE values are employed, namley specific IgE (sIgE) and total IgE minus specific IgE (tIgE - sIgE).

Thus, in a prefered embodiment, the invention relates to a method of assessing the allergic status of a subject to a specific allergen, the method comprising the steps of determining a subject-specific numeric value for a plurality of variables comprising age, sex, SPT, tIgE-sIgE, and allergic symptoms, applying a allergen-specific weighting to the numeric value for each variable, and correlating the weighted numeric values with risk of the subject being allergic to the allergan.

The method of the invention is suitable for use with any allergen, especially peanut, egg, and milk.

The weighted numeric values are suitably correlated with allergenicity status using a linear regression model P = ez/(1= ez) in which:

P = predicted allergic status for the subject;
e = mathematical constant (2.71828....); and
z = linear combination of the weighted numeric values for the variables.

Preferably, z = a linear combination x and β, in which x is the numercal value for a given variable, and β is the allergan-specific weighting (estimator) for that variable.
Thus, in a preferred embodiment:

$$P = [e(\alpha + \beta1x1 + \beta2x2 + .... + \beta qxq)] / [1 + e(\alpha + \beta1x1 + \beta2x2 + .... + \beta qxq)],$$

wherein α is a constant.
Typically, the numeric value for sex is 0 for a female and 1 for a male.
Suitably, the numeric values for allergic symptoms are 1, 2, 3 and 4, in which 1 correlates with skin or oral or gastrointestinal or upper respiratory symptoms , 2 correlates with upper respiratory and gastrointestinal symptoms (or two systems), 3 correlates with lower respiratory symptoms (or three systems), and 4 correlates with cardiovascular (or four systems).
SPT values are suitably provided in mm, sIgE values are typically provided in kUa/L, and tIgE-sIgE values are suitably provided in kUa. Age values are provided in years.
In a preferred embodiment, the peanut-specific weighting values (estimators) are selected from -0.37 for age, 4.6 for sex, 2.85 for STP, 0.5 for sIgE, -0.002 for tIgE-sIgE, and 3.32, 4.61, 7.86, and 11.08 for allergic symptoms numeric values 1, 2, 3, and 4, respectively.
In a preferred embodiment, the egg-specific weighting values (estimators) are selected from -0.15 for age, 1.7 for sex, 0.29 for STP, 0.52 for sIgE, -0.004 for tIgE-sIgE, and 1.4, 2.08, 2.74, and 3.76 for allergic symptoms numeric values 1, 2, 3, and 4, respectively.
In a preferred embodiment, the milk-specific weighting values (estimators) are selected from -0.15 for age, 0.59 for sex, 0.35 for STP, 1.8 for sIgE, -0.006 for tIgE-sIgE, and 0.48, 2.36, 7.92, and 8.51 for allergic symptoms numeric values 1, 2, 3, and 4, respectively.
It will be appreciated that the weighting values provided above are optimal weighting values and that weighting values within +/- 20%, preferably +/- 15%, more preferably within +/- 10% of the optimal weighting values, may be employed in the method of the invention. Variation on the weighting values may be employed to account for genetic variation in the population.
The algorithm provides a predictive value (P) between 0 and 1 for allergic status to a specific allergan for a subject. A predictive value (P) less than 0.5 indicates a negative allergic status to the allergen (i.e. negative prediction of allergy), and a predictive value of 0.5 or greater indicates a positive allergic status to the allergen (positive prediction of allergy). The algorithm may also be employed to predict population threshold values for specific allergans.
The invention also relates to a method of monitoring a course of immunotherapy in a subject, which method comprises a step of assessing the allergic status of the subject according to a method of any of the invention. Suitably, when the subject has a positive allergic status the immunotherapy is continued. Thus, the method of the invention may be employed to determine a stop/continue point during the maintenance phase in oral immunotherapy. Typically, when the subject has a negative allergic status, the immunotherapy is discontinued. In one embodiment, the method involves a step of determining the allergic status of the subject prior to the start of the course of therapy, and monitoring the allergic status of the subject periodically during the course of therapy to determine the effectiveness of the therapy.
The invention also relates to a method of assessing allergeic status in an individual to a specific allergen comprising a step of correlating a value for tIgE-sIgE for the subject with allergic status. Thus, for example, as the tIgE-sIgE value decreases, the subject has a decreased likelihood of a positive allergic status, and when the tIgE-sIgE value increases, the subject has an increased likelihood of a positive allergic status.
There is also provided a computer program comprising program instructions for causing a computer program to carry out the above method which may be embodied on a record medium, carrier signal or read-only memory. Thus, the methods of the invention may be carried out using a computer program, in which a user inputs numeric data relating to the subject-specific variables, and the computer program correlates the input data with allergic status. The computer program that is employed may be carried on a user's computer (locally), or may be accessed from a remote server via the internet.

<u>Detailed Description of the Invention</u>

The invention will be more clearly understood from the following description of some embodiments thereof given by way of example only.

**Variables**

The preferred implementation of this invention utilizes the following variables:

- Sex
- Age
- Grade of reaction
- sIgE
- Total IgE minus (-) sIgE
- SPT

**Grade of Reaction**

The grades for allergic symptoms are 1, 2, 3 and 4, in which:

- 1 correlates with skin or oral or gastrointestinal or upper respiratory symptoms;
- 2 correlates with upper respiratory and gastrointestinal symptoms (or two systems);
- 3 correlates with lower respiratory symptoms (or three systems); and
- 4 correlates with cardiovascular (or four systems).

A subject, for example, who is suspected of having peanut allergy and who has had a history of Grade 2 symptoms will have a higher odds ratio for a positive outcome than a subject with Grade 1 symptoms or with no symptoms, controlling for IgE result, SPT wheal diameter, sex, and age.

Food allergy occurs when the body's immune system mounts an exaggerated response against the offending food, which acts as an allergen. It is a type of hypersensitivity reaction.

In an IgE-mediated reaction, symptoms involving the oropharynx and gastrointestinal tract may occur within minutes of ingesting a food allergen. Itching and swelling of the lips, tongue, and soft palate may occur alone or in addition to gastrointestinal symptoms such as nausea, abdominal pain, vomiting, and diarrhea. Symptoms in the respiratory tract include wheezing or shortness of breath.

In summary, food allergy reactions can affect any of the four following areas of the body or systems:

**skin:** itchy red bumps (hives); eczema; redness and swelling of the face or extremities; itching and swelling of the lips, tongue, or mouth (skin reactions are the most common type of reaction)

**gastrointestinal tract:** abdominal pain, nausea, vomiting, or diarrhea

**respiratory tract:** runny or stuffy nose, sneezing, coughing, wheezing, shortness of breath

**cardiovascular system:** lightheadedness or fainting

A serious allergic reaction with widespread effects on the body is known as anaphylaxis. This sudden, potentially life-threatening allergic reaction involves two or more of the body areas listed above. In addition, there also can be swelling of the airway, serious difficulty with breathing, a drop in blood pressure, loss of consciousness, and in some cases, even death.

**SPT**

Skin prick testing was performed by using positive control (histamine dihydrochloride 10mg/mL), negative control (50% glycerol and 50% buffered saline), the potential food allergen (ALK-Abello, Reading UK), and single headed lancet. Skin reactions were recorded after 15 minutes. Positive test was a wheal of ≥3mm. The size of the wheal was determined as the mean of two perpendicular longest diameters. Serum specific IgE was measured by using the Unicap system (Phadia, Uppsala, Sweden) and results were reported in kUa/L.

**Estimators (B<sub>var</sub>)**

Estimators ($B_{var}$)

| Allergen | Indicators | b-estimate | p-value | Odds ratio | C.I. |
|---|---|---|---|---|---|
| **Peanut*** | | | | | |
| | Male | 4.60 | 0.03 | 96.4 | 46.1-128.5 |
| Grade of symptoms** | 1 | 3.32 | 0.01 | 27.5 | 15.9-46.2 |
| | 2 | 4.61 | 0.05 | 97.4. | 48.6-154.0 |
| | 3 | 7.86 | 0.04 | 231.5 | 136.7-365.3 |
| | 4 | 11.08 | 0.01 | 613.2 | 414.0-870.2 |
| | SPT (mm) | 2.85 | 0.01 | 16.9 | 10.4-24.6 |
| | sIgE (kUa/L) | 0.50 | 0.04 | 1.6 | 1.2-2.0 |
| | IgE-sIgE (kUa) | -0.002 | 0.01 | 1.2 | 1.0-1.4 |
| | Age (years) | -0.37 | 0.05 | 0.69 | 0.3-0.9 |
| | Constant | -11.63 | 0.01 | | |
| **Egg*** | | | | | |
| | Male | 1.70 | 0.04 | 5.5 | 3.5-8.0 |
| Grade of symptoms** | 1 | 1.40 | 0.01 | 4.0 | 2.3-10.3 |
| | 2 | 2.08 | 0.03 | 8.1 | 6.6-11.3 |
| | 3 | 2.74 | 0.01 | 15.2 | 10.4-18.9 |
| | 4 | 3.76 | 0.02 | 43.5 | 33.2-68.0 |
| | SPT(mm) | 0.29 | 0.01 | 1.30 | 1.1-3.1 |
| | IgE-sIgE (kUa) | -0.004 | 0.04 | 1.21 | 1.0-1.3 |
| | sIgE (kUa/L) | 0.20 | 0.01 | 1.22 | 1.1-1.7 |
| | Age (years) | -0.15 | 0.03 | 0.80 | 0.5-0.9 |
| | Constant | -2.42 | 0.01 | | |
| **Milk*** | | | | | |
| | Male | 0.59 | 0.05 | 1.81 | 1.3-2.5 |
| Grade of symptoms** | 1 | 0.48 | 0.05 | 1.62 | 1.4-1.8 |
| | 2 | 2.36 | 0.03 | 3.01 | 1.8-4.6 |
| | 3 | 7.92 | 0.02 | 25.50 | 18.1-393.0 |
| | 4 | 8.51 | 0.02 | 46.4 | 27.6-66.3 |
| | SPT (mm) | 0.35 | 0.05 | 1.41 | 1.0-2.4 |
| | IgE-sIgE (kUa) | -0.006 | 0.01 | 1.3 | 1.1-4.2 |
| | sIgE (kUa/L) | 1.8 | 0.01 | 5.9 | 3.9-10.1 |
| | Age (years) | -0.15 | 0.05 | 0.86 | 0.3-0.9 |

(continued)

| Milk* | | | | | |
|---|---|---|---|---|---|
| | Constant | -14.61 | 0.02 | | |
| Data from Group B. * Food being challenged. ** Each of the 4 levels is has its own estimator with no symptoms (0) as the indicator. 1= skin or oral or gastrointestinal or upper respiratory only; 2= upper respiratory and gastrointestinal or 2 systems; 3 = lower respiratory or 3 systems; 4= cardiovascular or 4 systems. | | | | | |

Multivariate regression analyses with data presented are b-estimates with P-value, odds ratio (OR) and confidence intervals (CI).

**Correlating variable values with risk**

The values obtained for the variables are weighted using the above-referenced allergen-specific estimators, and correlated with allergenicity status using the following algorithm:

$$PFA = [\exp(\text{constant}+ B_{sex}{}^*sex + B_{symptoms} + B_{SPT}{}^*SPT + B_{sIgE}{}^*sIgE + B \times (\text{total IgE - sIgE}) +$$

$$B \times age] / [1+ \exp \text{constant}+ B \times sex + B (\text{symptoms grade}) + B \times SPT \text{ level} + B \times sIgE + B$$

$$\times (\text{total IgE - sIgE}) + B \times age].$$

Where probability of food allergy diagnosis based on challenge is PFA

Example 1 : Suspected Peanut Allergy

Male, 3 years of age, total IgE (-) sIgE of 1772 units, SPT level of 5 mm, sIgE of 10 KUa/L, a reaction history of skin swelling, vomiting and cough.

$$[\exp (-11.63 + (4.60 \times 1) + 11.08 + (5 \times 2.85) + (10 \times 0.50) + (1872 \times -0.002) + (3$$

$$\times -0.37))] / [1+ \exp (-11.63 + (4.60 \times 1) + 11.08 + (5 \times 2.85) + (10 \times 0.50) + (1872$$

$$\times -0.002) + (3 \times -0.37))]$$

The probability of a positive outcome is 1.0 for this child.

Example 2 : Suspected Milk Allergy

Male, 12 months of age, total IgE (-) sIgE of 170 units, SPT level of 6 mm, sIgE of 12 KUa/L, a reaction history of vomit, wheeze and eczema.

$$[\exp (-14.61 + (0.59 \times 1) + 7.92 + (6 \times 0.35) + (12 \times 1.8) + (170 \times -0.006) + 1 \times -$$

$$0.15)] / [1+ \exp (-14.61 + (0.59 \times 1) + 7.92 + (6 \times 0.35) + (12 \times 1.8) + (170 \times -$$

$$0.006) + 1 \times -0.15)]$$

The probability of a positive outcome is 1.0 for this child.

Example 3 : Suspected Egg Allergy

Female, 10 years of age, total IgE (-) sIgE of 1650 units, SPT level of 3 mm, sIgE of 9 KUa/L, and no previous history of reactions.

$$[\exp(-2.42 + (1.7 \times 0) + (3 \times 0.29) + (9 \times 0.20) + (1650 \times -0.004) + (10 \times -0.15)] /$$

$$[1+ \exp(-2.42 + (1.7 \times 0) + (3 \times 0.29) + (9 \times 0.20) + (1650 \times -0.004) + (10 \times -0.15)]$$

The probability of a positive outcome is 0.0004 for this child.

Example 4 : Suspected Peanut Allergy

Female, 8 years of age, total IgE (-) sIgE of 250 units, SPT level of 4 mm, sIgE of 5.19 KUa/L, and no previous history of reactions.

$$[\exp(-11.63 + (250 \times -0.002) + (4 \times 2.846) + (4.60 \times 0) + (5.19 \times 0.50) + (8 \times -0.37)] / [1+ (-11.63 + (250 \times -0.002) + (4 \times 2.846) + (4.60 \times 0) + (5.19 \times 0.50) + (8 \times -0.37)]$$

The probability of a positive outcome is 0.251 for this child.

Example 5

The method of the invention was employed to predict allergenicity in a cohort of patients described in Table 1. Known methods of predicting allergenicity were also employed on the cohort. The outcome of the various methods of predicting are shown in Table 2.

**Table 1:** Demographic and clinical characteristics of group C.

|  | Group C (N=70) |
|---|---|
| Males | 50 (71%) |
| Age (Mean) | 8 (3.0) |
| History of reaction | 39 (56%) |
| **Peanuts (%)** | 30 (42%) |
| SPT level (Mean) | 5.4 (2.1) |
| sIgE level (Mean) | 8.2 (10.5) |
| Total IgE (Mean) | 1129 (842) |
| **Milk (%)** | 20 (29%) |
| SPT level (Mean) | 5.0 (2) |
| sIgE level (Mean) | 8.7 (12) |
| Total IgE (Mean) | 1043 (1166) |
| **Egg (%)** | 20 (29%) |
| SPT level (Mean) | 5.2 mm (2.5) |
| sIgE level (Mean) | 8.1 kUa/L (10) |
| Total IgE (Mean) | 1209 kU (983) |
| No previous history | 31 (44%) |
| Skin or oral or gastrointestinal or upper respiratory only | 11 (16%) |
| Upper respiratory and gastrointestinal or 2 systems | 17 (24%) |
| Lower respiratory or 3 systems | 8 (12%) |
| Cardiovascular or 4 systems | 3 (4%) |

(continued)

| Diagnosis Positive | |
| --- | --- |
| Peanuts | 52 (74%) |
| Milk | 49 (71%) |
| Egg | 51 (73%) |

**Table 2:** Data from Group C (N = 70).

| Diagnostic method | Sensitivity | Specificity | Accuracy |
| --- | --- | --- | --- |
| sIgE only | 75% | 45% | **61%** |
| SPT only | 85% | 65% | **75%** |
| sIgE & SPT | 87% | 75% | **81%** |
| Present Invention | 97% | 96% | **96%** |

The data in Table 2 shows that the method of the invention provides superior sensitivity and specificity compared to the sIgE, SPT, and sIgE and SPT, methods of prediction.

Example 6

In another embodiment, only 5 variables are used to predict food test outcome, these are:

- Sex
- Age
- Grade of reaction
- sIgE
- SPT

These are used in the following equation:

$$PFA = [\exp(\text{constant} + B_{sex}{}^{*}\text{sex} + B_{symptoms} + B_{SPT}{}^{*}\text{SPT} + B_{IgE}{}^{*}\text{sIgE} + B \times \text{age})] / [1 + \exp(\text{constant} + B_{sex}{}^{*}\text{sex} + B_{symptoms} + B_{SPT}{}^{*}\text{SPT} + B_{IgE}{}^{*}\text{sIgE} + B \times \text{age})],$$

where probability of food allergy diagnosis based on challenge is PFA

No allergen specific modification was made to the algorithm's coefficients on an allergen by allergen basis.

With a test set of 429 as described by Table 3, this model (with coefficients from Table 4) had 80% accuracy in correctly predicting positive outcomes and 75% accuracy in negative outcomes.

**Table 3:** Demographic and clinical characteristics of group A.

| | Group A (N=429) |
| --- | --- |
| Males | 283 (66%) |
| Age (Mean) | 7 (3.7) |
| History of reaction | 292 (68%) |
| **Peanuts (%)** | 239 (56%) |
| SPT level (Mean) | 4.0 (2.2) |
| sIgE level (Mean) | 5.8 (4.0) |
| Total IgE (Mean) | - |
| **Milk (%)** | 110 (26%) |
| SPT level (Mean) | 3.7 (2.8) |
| sIgE level (Mean) | 6.0 (4.1) |
| Total IgE (Mean) | - |
| **Egg (%)** | 80 (18%) |

(continued)

| | Group A (N=429) |
|---|---|
| SPT level (Mean) | 3.8 mm (3.1) |
| sIgE level (Mean) | 5.7 kUa/L(3.6) |
| Total IgE (Mean) | |
| No previous history | 137 (32 %) |
| Skin or oral or gastrointestinal or upper respiratory only | 82 (19%) |
| Upper respiratory and gastrointestinal or 2 systems | 137 (32%) |
| Lower respiratory or 3 systems | 60 (14%) |
| Cardiovascular or 4 systems | 13 (3%) |
| **Diagnosis Positive** | |
| Peanuts | 163 (38%) |
| Milk | 172 (40%) |
| Egg | 167 (39%) |

**Table 4:** Estimators for all foods.

| Indicators | b-estimate | p-value | Odds ratio | C.I. |
|---|---|---|---|---|
| High SPT * | 4.10 | .050 | 8.18 | 4.5-11.2 |
| High IgE ** | 2.26 | .045 | 3.54 | 2.1-5.8 |
| Male | 1.55 | .033 | 2.86 | 1.9-4.4 |
| Age <7years | 1.50 | .050 | 1.43 | 1.1-3.9 |
| History of reaction | 2.40 | .044 | 1.49 | 1.2- 3.7 |
| Constant | -3.07 | .007 | | |
| *≥8mm (peanut and milk), ≥7mm (egg) **≥15 kUa/L (peanut and milk), ≥7 kUa/L (egg). | | | | |

The invention is not limited to the embodiments hereinbefore described which may be varied in construction, detail, and process step, without departing from the spirit of the invention.

**Claims**

1. A method of assessing the allergic status of a subject to a specific allergen, the method comprising the steps of determining a subject-specific numeric value for a plurality of variables comprising age, sex, SPT, IgE, and allergic symptoms, applying an allergen-specific weighting to the numeric value for each variable, and correlating the weighted numeric values with allergic status for the subject.

2. A method as claimed in Claim 1 in which at least six variables are employed including age, sex, SPT, tIgE minus sIgE, and allergic symptoms.

3. A method as claimed in Claim 1 in which the weighted numeric values are correlated with allergenicity status using a linear regression model $P = e^z/(1= e^z)$ in which:

   P = predicted allergenicity status for the subject;
   e = mathematical constant (2.71828....); and
   z = linear combination of the weighted numeric values for the variables.

4. A method as claimed in Claim 3 in which z = a linear combination x and $\beta$, in which x is the numercal value for a given variable, and $\beta$ is the allergen-specific weighting (estimator) for that variable.

5. A method as claimed in Claim 3 and 4 in which the weighted numeric values are correlated with allergenicity status using a linear regression model: $P = [e(\alpha + \beta_1 x_1 + \beta_2 x_2 + .... + \beta_q x_q)] / [1 + e(\alpha + \beta_1 x_1 + \beta_2 x_2 + .... + \beta_q x_q)]$, wherein

α is a constant.

6. A method as claimed in any preceding Claim in which the numeric value for sex is 0 for a female and 1 for a male.

7. A method as claimed in any preceding Claim in which the numeric values for allergic symptoms are 1, 2, 3 and 4, in which 1 correlates with skin or oral or gastrointestinal or upper respiratory symptoms , 2 correlates with upper respiratory and gastrointestinal symptoms (or two systems), 3 correlates with lower respiratory symptoms (or three systems), and 4 correlates with cardiovascular (or four systems).

8. A method as claimed in any preceding Claim in which the peanut-specific weighting values (estimators) are selected from -0.37 for age, 4.6 for sex, 2.85 for STP, 0.5 for sIgE, -0.002 for tIgE-sIgE, and 3.32, 4.61, 7.86, and 11.08 for allergic symptoms numeric values 1, 2, 3, and 4, respectively.

9. A method as claimed in any preceding Claim in which the egg-specific weighting values (estimators) are selected from -0.15 for age, 1.7 for sex, 0.29 for STP, 0.52 for sIgE, -0.004 for tIgE-sIgE, and 1.4, 2.08, 2.74, and 3.76 for allergic symptoms numeric values 1, 2, 3, and 4, respectively.

10. A method as claimed in any preceding Claim in which the milk-specific weighting values (estimators) are selected from -0.15 for age, 0.59 for sex, 0.35 for STP, 1.8 for sIgE, -0.006 for tIgE-sIgE, and 0.48, 2.36, 7.92, and 8.51 for allergic symptoms numeric values 1, 2, 3, and 4, respectively.

11. A method of monitoring a course of immunotherapy in a subject, which method comprises a step of assessing the allerginicity status of the subject according to a method of any of Claims 1 to 10 at least once during the course of the immunotherapy.

12. A method as claimed in Claim 11 for use in determining a stop/continue point during the maintenance phase in oral immunotherapy.

13. A computer program comprising program instructions for causing a computer program to carry out a method of any of Claims 1 to 12 which may be embodied on a record medium, carrier signal or read-only memory.

Europäisches
Patentamt

European
Patent Office

Office européen
des brevets

# EUROPEAN SEARCH REPORT

Application Number

EP 10 16 3132

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | SEYMOUR D G ET AL:  "Making Better Decisions: Construction of Clinical Scoring Systems by the Spielgelhalter: Knill-Jones Approach" BRITISH MEDICAL JOURNAL, LONDON, GB, vol. 300, 27 January 1990 (1990-01-27), pages 223-226, XP009139940 ISSN: 0267-0623 * the whole document * | 1-13 | INV. G06F19/00 |
| X | US 2008/082364 A1 (JUNG EDWARD K [US] ET AL) 3 April 2008 (2008-04-03) * claim 48; figure 13 * * paragraph [0039] * * paragraph [0059] * * paragraph [0152] - paragraph [0153] * * paragraph [0228] * | 1-13 | |
| X | KEARNEY S ET AL:  "The Food Challenge Risk Index: Predicting Positive Open Food Challenges to Milk, Egg and Peanuts in Children" PEDIATRIC ASTHMA, ALLERGY & IMMUNOLOGY, vol. 18, no. 2, 1 June 2005 (2005-06-01), pages 68-76, XP002606680 * the whole document * | 1-13 | TECHNICAL FIELDS SEARCHED (IPC) G06F |

The present search report has been drawn up for all claims

| Place of search | Date of completion of the search | Examiner |
|---|---|---|
| Munich | 27 October 2010 | Sisk, Aisling |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 10 16 3132

This annex lists the patent family members relating to the patent documents cited in the above-mentioned European search report.
The members are as contained in the European Patent Office EDP file on
The European Patent Office is in no way liable for these particulars which are merely given for the purpose of information.

27-10-2010

| Patent document cited in search report | Publication date | Patent family member(s) | Publication date |
|---|---|---|---|
| US 2008082364 A1 | 03-04-2008 | NONE | |

For more details about this annex : see Official Journal of the European Patent Office, No. 12/82